(19)

Europäisches Patentamt
European Patent Office
Office européen des brevets

(11)  **EP 3 341 125 B1**

(12)  # EUROPEAN PATENT SPECIFICATION

(45) Date of publication and mention
of the grant of the patent:
**09.10.2024  Bulletin 2024/41**

(21) Application number: **16766717.9**

(22) Date of filing: **29.08.2016**

(51) International Patent Classification (IPC):
**B01J 27/18** *(2006.01)*       **C07C 51/377** *(2006.01)*
**B01J 27/182** *(2006.01)*      **B01J 27/186** *(2006.01)*
**B01J 37/00** *(2006.01)*       **B01J 23/00** *(2006.01)*
**B01J 37/04** *(2006.01)*       **C07C 67/327** *(2006.01)*
**B01J 35/30** *(2024.01)*       **B01J 35/40** *(2024.01)*

(52) Cooperative Patent Classification (CPC):
(C-Sets available)
**C07C 51/377; B01J 23/007; B01J 27/1806;**
**B01J 27/1811; B01J 27/1813; B01J 27/1815;**
**B01J 27/182; B01J 27/186; B01J 35/30;**
**B01J 35/40; B01J 37/0036; B01J 37/009;**
**B01J 37/04; C07C 67/327;** B01J 2523/00    (Cont.)

(86) International application number:
**PCT/US2016/049225**

(87) International publication number:
**WO 2017/040386 (09.03.2017 Gazette 2017/10)**

(54) **CATALYTIC DEHYDRATION OF HYDROXYPROPIONIC ACID AND ITS DERIVATIVES**

KATALYTISCHE DEHYDRIERUNG VON HYDROXYPROPIONSÄURE UND DEREN DERIVATEN

DÉSHYDRATATION CATALYTIQUE D'ACIDE HYDROXYPROPIONIQUE ET DE SES DÉRIVÉS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **28.08.2015   US 201562211015 P**

(43) Date of publication of application:
**04.07.2018   Bulletin 2018/27**

(73) Proprietor: **The Procter & Gamble Company
Cincinnati, OH 45202 (US)**

(72) Inventors:
 • **VELASQUEZ, Juan Esteban
   Cincinnati, Ohio 45202 (US)**
 • **COLLIAS, Dimitris Ioannis
   Cincinnati, Ohio 45202 (US)**
 • **GODLEWSKI, Jane Ellen
   Cincinnati, Ohio 45202 (US)**
 • **WIREKO, Fred Christian
   Cincinnati, OH 45202 (US)**

(74) Representative: **P&G Patent Germany
Procter & Gamble Service GmbH
Sulzbacher Straße 40
65824 Schwalbach am Taunus (DE)**

(56) References cited:
**US-A- 2 859 240        US-A1- 2013 274 515
US-A1- 2015 105 584**

• **JINFENG ZHANG ET AL: "Catalytic dehydration
of lactic acid to acrylic acid over sulfate
catalysts", CANADIAN JOURNAL OF CHEMICAL
ENGINEERING, vol. 86, no. 6, 1 December 2008
(2008-12-01), US, CA, pages 1047 - 1053,
XP055671110, ISSN: 0008-4034, DOI:
10.1002/cjce.20115**

**(Cont. next page)**

- ZHANG J ET AL: "Evaluation of Catalysts and Optimization of Reaction Conditions for the Dehydration of Methyl Lactate to Acrylates", CHINESE JOURNAL OF CHEMICAL ENGINEERING, CHEMICAL INDUSTRY PRESS, BEIJING, CN, vol. 16, no. 2, 1 April 2008 (2008-04-01), pages 263 - 269, XP022856567, ISSN: 1004-9541, [retrieved on 20080401], DOI: 10.1016/S1004-9541(08)60073-7
- YU-KI TANINOUCHI ET AL: "Phase Relationship of CsH[sub 2]PO[sub 4]-CsPO[sub 3] System and Electrical Properties of CsPO[sub 3]", JOURNAL OF THE ELECTROCHEMICAL SOCIETY, vol. 156, no. 5, 1 January 2009 (2009-01-01), US, pages B572, XP055315501, ISSN: 0013-4651, DOI: 10.1149/1.3086755
- AYAKO IKEDA ET AL: "The thermodynamics and kinetics of the dehydration of CsH2PO4 studied in the presence of SiO2", SOLID STATE IONICS, vol. 213, 1 April 2012 (2012-04-01), NL, pages 63 - 71, XP055315503, ISSN: 0167-2738, DOI: 10.1016/j.ssi.2011.09.018

(52) Cooperative Patent Classification (CPC): (Cont.)

C-Sets
**C07C 51/377, C07C 57/04;**
**C07C 67/327, C07C 69/54;**
B01J 2523/00, B01J 2523/13, B01J 2523/25,
B01J 2523/41, B01J 2523/51, B01J 2523/62

## Description

FIELD OF THE INVENTION

The present invention is defined by the appended claims and generally relates to methods for catalytic dehydration of hydroxypropionic acid, hydroxypropionic acid derivatives, or mixtures thereof to acrylic acid, acrylic acid derivatives, or mixtures thereof with high yield and selectivity to acrylic acid, acrylic acid derivatives, or mixtures thereof, short residence time, and without significant conversion of the hydroxypropionic acid, hydroxypropionic acid derivatives, or mixtures thereof to undesired side products, such as, for example, acetaldehyde, propionic acid, acetic acid, 2,3-pentanedione, carbon dioxide, and carbon monoxide.

BACKGROUND OF THE INVENTION

Acrylic acid, acrylic acid derivatives, or mixtures thereof have a variety of industrial uses, typically consumed in the form of polymers. In turn, these polymers are commonly used in the manufacture of, among other things, adhesives, binders, coatings, paints, polishes, detergents, flocculants, dispersants, thixotropic agents, sequestrants, and superabsorbent polymers (SAP), which are used in disposable absorbent articles, comprising diapers and hygienic products, for example. Acrylic acid is commonly made from petroleum sources. For example, acrylic acid has long been prepared by catalytic oxidation of propylene. These and other methods of making acrylic acid from petroleum sources are described in the Kirk-Othmer Encyclopedia of Chemical Technology, Vol. 1, pgs. 342 - 369 (5th Ed., John Wiley & Sons, Inc., 2004). As petrochemical resources become increasingly scarce, more expensive, and subject to regulations for $CO_2$ emissions, there exists a growing need for bio-based acrylic acid, acrylic acid derivatives, or mixtures thereof that can serve as an alternative to petroleum-based acrylic acid, acrylic acid derivatives, or mixtures thereof.

Many attempts have been made over the last 80 years to make bio-based acrylic acid, acrylic acid derivatives, or mixtures thereof from non-petroleum sources, such as lactic acid (also known as 2-hydroxypropionic acid), lactic acid derivatives (e.g. alkyl 2-acetoxy-propionate and 2-acetoxy propionic acid), 3-hydroxypropionic acid, glycerin, carbon monoxide and ethylene oxide, carbon dioxide and ethylene, and crotonic acid. From these non-petroleum sources, only lactic acid is produced today in high yield from sugar ($\geq$ 90% of theoretical yield, or equivalently, $\geq$ 0.9 g of lactic acid per g of sugar). Furthermore, commercial lactic acid purity and economics could favor producing acrylic acid at a cost competitive to petroleum-based acrylic acid. As such, lactic acid or lactate presents a real opportunity of serving as a feedstock for bio-based acrylic acid, acrylic acid derivatives, or mixtures thereof. Also, 3-hydroxypropionic acid is expected to be produced at commercial scale in a few years, and as such, 3-hydropropionic acid will present another real opportunity of serving as feedstock for bio-based acrylic acid, acrylic acid derivatives, or mixtures thereof. Sulfate salts, phosphate salts, mixtures of sulfate and phosphate salts, bases, zeolites or modified zeolites, metal oxides or modified metal oxides, and supercritical water are the main catalysts which have been used to dehydrate lactic acid or lactate to acrylic acid, acrylic acid derivatives, or mixtures thereof in the past with varying success.

For example, U.S. Patent No. 4,786,756 (issued in 1988), describes the vapor phase dehydration of lactic acid or ammonium lactate to acrylic acid using aluminum phosphate ($AlPO_4$) treated with an aqueous inorganic base as a catalyst. As an example, the '756 patent discloses a maximum yield of acrylic acid of 43.3% when lactic acid was fed into the reactor at approximately atmospheric pressure, and a respective yield of 61.1% when ammonium lactate was fed into the reactor. In both examples, acetaldehyde was produced at yields of 34.7% and 11.9%, respectively, and other side products were also present in large quantities, such as propionic acid, CO, and $CO_2$. Omission of the base treatment caused increased amounts of the side products. Another example is Hong et al., Appl. Catal. A: General 396:194-200 (2011), who developed and tested composite catalysts made with $Ca_3(PO_4)_2$ and $Ca_2(P_2O_7)$ salts with a slurry-mixing method. The catalyst with the highest yield of acrylic acid from methyl lactate was the 50%-50% (by weight) catalyst. It yielded 68% acrylic acid, about 5% methyl acrylate, and about 14% acetaldehyde at 390°C. The same catalyst achieved 54% yield of acrylic acid, 14% yield of acetaldehyde, and 14% yield of propionic acid from lactic acid.

Prof. D. Miller's group at Michigan State University (MSU) published many papers on the dehydration of lactic acid or lactic acid esters to acrylic acid and 2,3-pentanedione, such as Gunter et al., J. Catalysis 148:252-260 (1994); and Tam et al., Ind. Eng. Chem. Res. 38:3873-3877 (1999). The best acrylic acid yields reported by the group were about 33% when lactic acid was dehydrated at 350 °C over low surface area and pore volume silica impregnated with NaOH. In the same experiment, the acetaldehyde yield was 14.7% and the propionic acid yield was 4.1%. Examples of other catalysts tested by the group were $Na_2SO_4$, NaCl, $Na_3PO_4$, $NaNO_3$, $Na_2SiO_3$, $Na_4P_2O_7$, $NaH_2PO_4$, $Na_2HPO_4$, $Na_2HAsO_4$, $NaC_3H_5O_3$, NaOH, CsCl, $Cs_2SO_4$, KOH, CsOH, and LiOH. In all cases, the above referenced catalysts were tested in gas phase reactions with low partial pressures of water, as commonly suggested in the art for dehydration reactions. Finally, the group suggested that the acrylic acid yield is increased (and the by-product yields are decreased) when the surface area of the silica support is low, the reaction temperature is high, the reaction pressure is low, and the residence time of the reactants in the catalyst bed is short.

Finally, the Chinese patent application 200910054519.7 discloses the use of ZSM-5 molecular sieves modified with aqueous alkali (such as $NH_3$, NaOH, and $Na_2CO_3$) or a phosphoric acid salt (such as $NaH_2PO_4$, $Na_2HPO_4$, $LiH_2PO_4$, $LaPO_4$, etc.). The best yield of acrylic acid achieved in the dehydration of lactic acid was 83.9%, however that yield came at very long residence times.

Therefore, the manufacture of acrylic acid, acrylic acid derivatives, or mixtures thereof from lactic acid or lactate by processes, such as those described in the literature noted above, has demonstrated: 1) yields of acrylic acid, acrylic acid derivatives, or mixtures thereof not exceeding 70% at short residence times; 2) low selectivities of acrylic acid, acrylic acid derivatives, or mixtures thereof, i.e., significant amounts of undesired side products, such as acetaldehyde, 2,3-pentanedione, propionic acid, CO, and $CO_2$; 3) long residence times in the catalyst beds; and 4) catalyst deactivation in short time on stream (TOS). The side products can deposit onto the catalyst resulting in fouling, and premature and rapid deactivation of the catalyst. Further, once deposited, these side products can catalyze other undesired reactions. Aside from depositing on the catalysts, these side products, even when present in only small amounts, impose additional costs in processing acrylic acid (when present in the reaction product effluent) towards the manufacture of SAP, for example. These deficiencies of the prior art processes and catalysts render them commercially non-viable.

Accordingly, there is a need for catalysts, methods of making the catalysts, and processes for the dehydration of hydroxypropionic acid, hydroxypropionic acid derivatives, or mixtures thereof to acrylic acid, acrylic acid derivatives, or mixtures thereof, with high yield and selectivity toward acrylic acid, in an efficient manner (i.e. short residence times), and with suitable catalyst longevity.

US 2013/274515 A1 discusses bio-based glacial acrylic acid, produced from hydroxypropionic acid.

US 2 859 240 A discusses catalytically producing acrylate material from lactate material which comprises the steps of (I) bringing volatile lactate into contact with a dehydration catalyst consisting of sulfates and phosphates of metals of groups I and II.

US 2015/105584A1 discuss dehydrating lactic acid derivatives using a catalyst and process to produce bio-acrylic acid, acrylic acid derivatives, or mixtures thereof.

Jinfeng Zhang ET AL: "Catalytic dehydration of lactic acid to acrylic acid over sulfate catalysts", Canadian Journal of Chemical Engineering, vol. 86, no. 6, 1 December 2008, p 1047-1053, XP055671110, ISSN: 0008-4034, DOI: 10.1002/cjce.20115, discusses catalytic dehydration of lactic acid to acrylic acid over sulfate catalysts based on a combination of m(CaSO4) / m(CuSO4) / m(Na2HPO4) / m(KH2PO4).

ZHANG J ET AL: "Evaluation of Catalysts and Optimization of Reaction Conditions for the Dehydration of Methyl Lactate to Acrylates", CHINESE JOURNAL OF CHEMICAL ENGINEERING,CHEMICAL INDUSTRY PRESS, vol. 16, no. 2, 1 April 2008, p 263-269, XP022856567, ISSN: 1004-9541, DOI: 10.1 016/S1 004-9541 (08)60073-7, discusses catalytic dehydration of lactic acid to acrylic acid over sulfate catalysts based on a combination of m(CaSO4) / m(CuSO4) / m(Na2HPO4) / m(KH2PO4).

SUMMARY OF THE INVENTION

The present invention provides a method in accordance with the claims.

BRIEF DESCRIPTION OF THE DRAWINGS

For a more complete understanding of the disclosure, reference should be made to the following detailed description and drawing Figures.

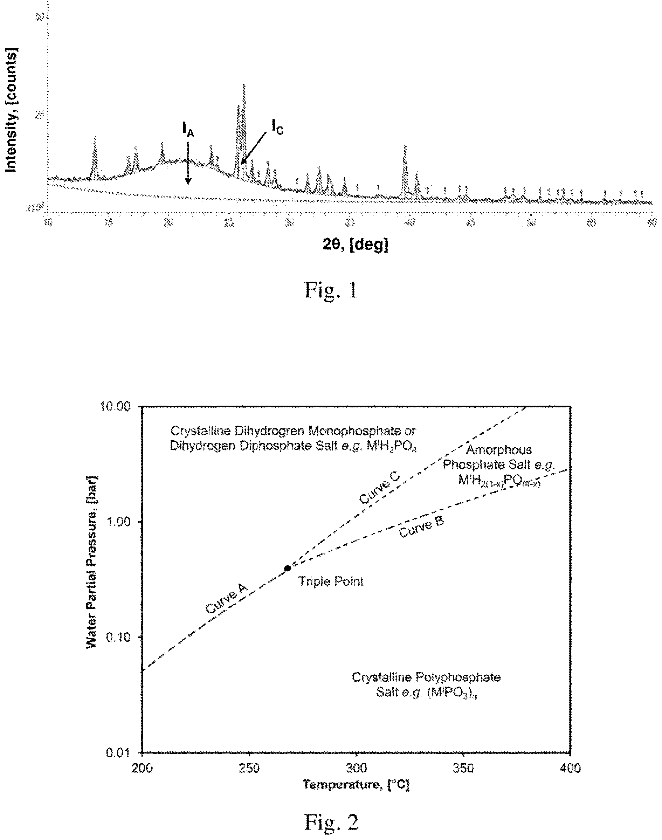

FIG. **1** illustrates the calculation of amorphous content in the dehydration catalyst using an XRD technique. The separate amorphous ($I_A$) and crystalline ($I_C$) contributions to the scattering pattern are determined using a profile-fitting technique, after appropriate background subtraction.
FIG. **2** illustrates a typical water partial pressure versus temperature phase equilibrium diagram of a dehydration catalyst (amorphous phosphate salt) and its precursor phosphate salts (crystalline phosphate salts). The triple point is located in the interception of the three phase boundary curves. $M^I$ is a monovalent cation. The reported values of water partial pressure are only an illustration and do not represent the real values for every specific dehydration catalyst described in the current invention.

While the disclosed catalysts and methods are susceptible of embodiments in various forms, there are illustrated in the figures (and will hereafter be described) specific embodiments of the invention, with the understanding that the disclosure is intended to be illustrative, and is not intended to limit the invention to the specific embodiments described and illustrated herein.

DETAILED DESCRIPTION OF THE INVENTION

I. Definitions

As used herein, the term "catalyst" refers to either a pre-reaction catalyst (also called a catalyst precursor mixture) or an *in-situ* catalyst. The pre-reaction catalyst is the catalyst loaded into the chemical reactor, and the *in-situ* catalyst is the catalyst present in the reactor during the reaction. In general, a catalyst increases the reaction rate without being consumed in the reaction. Finally, a pre-reaction catalyst can remain unchanged during the reaction or undergo *in-situ* physical or chemical transformations during the reaction that can change its physical and chemical properties and become an *in-situ* catalyst.

As used herein, the term "monophosphate" or "orthophosphate" refers to any salt whose anionic entity, $[PO_4]^{3-}$, is composed of four oxygen atoms arranged in an almost regular tetrahedral array about a central phosphorus atom.

As used herein, the term "condensed phosphate" refers to any salts containing one or several P-O-P bonds generated by corner sharing of $PO_4$ tetrahedra.

As used herein, the term "polyphosphate" refers to any condensed phosphates with a linear structure; i.e. containing linear P-O-P linkages by corner sharing of $PO_4$ tetrahedra leading to the formation of finite chains.

As used herein, the term "cyclophosphate" refers to any condensed phosphate with a cyclic structure.

As used herein, the term "hydrated" refers to a hydrated crystalline salt or hydrated crystalline compound that contains a specific number of water molecules per formula unit of the salt or compound.

As used herein, the term "monovalent cation" refers to any cation with a positive charge of +1.

As used herein, the term "polyvalent cation" refers to any cation with a positive charge equal or greater than +2.

As used herein, the term "anion" refers to any atom or group of covalently-bonded atoms having a negative charge.

As used herein, the term "heteropolyanion" refers to any anion with covalently bonded $XO_p$ and $YO_r$ polyhedra, and thus comprises X-O-Y and possibly X-O-X and Y-O-Y bonds, wherein X and Y represent any atoms, and wherein p and r are any positive integers.

As used herein, the term "heteropolyphosphate" refers to any heteropolyanion, wherein X represents phosphorus (P) and Y represents any other atom.

As used herein, the term "phosphate adduct" refers to any compound with one or more phosphate anions and one or more non-phosphate anions that are not covalently linked.

As used herein, the term "amorphous" refers to the state of any condensed phase material that lacks the long-range order characteristic of a crystalline material. An amorphous material can be either an amorphous solid or a liquid. In the context of the present invention, materials with more than 50 wt% of amorphous content are considered amorphous materials.

As used herein, the term "crystalline" refers to the state of any condensed phase material whose constituents are arranged in a highly ordered microscopic structure, forming a crystal lattice with long-range order. In the context of the present invention, materials with less than 50 wt% of amorphous content are considered crystalline materials.

As used herein, the term "chemically inert" materials refers to materials which remain in the same chemical form, under equilibrium conditions, when contacted with another material or materials. In the context of the present invention, more than about 90 wt% of the material should remain in the same chemical form to be considered a "substantially chemically inert" material and more than about 98 wt% of the material should remain in the same chemical form to be considered an "essentially chemically inert" material.

As used herein, the term "antioxidant" refers to a molecule capable of terminating radical chain processes by either donating a hydrogen atom or the reaction of an olefinic bond to form a stabilized organic radical and thus terminate radical chain processes. Non limiting examples of antioxidants comprise thiols, polyphenols, butylated hydroxy toluene (BHA), and butylated hydroxy anisole (BHA).

As used herein, the terms "LA" refers to lactic acid, "AA" refers to acrylic acid, "AcH" refers to acetaldehyde, and "PA" refers to propionic acid.

As used herein, the term "conversion" in % is defined as [hydroxypropionic acid, hydroxypropionic acid derivatives, or mixtures thereof flow rate in (mol/min) - hydroxypropionic acid, hydroxypropionic acid derivatives, or mixtures thereof flow rate out (mol/min)] / [hydroxypropionic acid, hydroxypropionic acid derivatives, or mixtures thereof flow rate in (mol/min)] x100. For the purposes of this invention, the term "conversion" means molar conversion, unless otherwise noted.

As used herein, the term "yield" in % is defined as [product flow rate out (mol/min) / hydroxypropionic acid, hydroxypropionic acid derivatives, or mixtures thereof flow rate in (mol/min)] x 100. For the purposes of this invention, the term "yield" means molar yield, unless otherwise noted.

As used herein, the term "selectivity" in % is defined as [Yield / Conversion] x 100. For the purposes of this invention, the term "selectivity" means molar selectivity, unless otherwise noted.

As used herein, the term "total carbon balance" is defined as: [((mol carbon monoxide out + mol carbon dioxide out + mol methane out) + (2 x (mol acetic acid out + mol acetaldehyde out + mol ethane out + mol ethylene out)) + (3 x (mol acrylic acid out + mol propionic acid out + mol hydroxypropionic acid out + mol hydroxyacetone out) + (5 x mol 2,3 pentanedione out) + (6 x mol acrylic acid dimer out)) / (3 x mol hydroxypropionic acid in)] x 100. If hydroxypropionic acid derivative is used instead of hydroxypropionic acid, the above formula needs to be adjusted according to the number of carbon atoms in the hydroxypropionic acid derivative.

As used herein, the term "Gas Hourly Space Velocity" or "GHSV" in $h^{-1}$ is defined as 60 x [Total gas flow rate (mL/min) / catalyst empty bed volume (mL)]. The total gas flow rate is calculated under Standard Temperature and Pressure conditions (STP; 0°C and 1 atm).

As used herein, the term "Weight Hourly Space Velocity" or "WHSV" in $h^{-1}$ is defined as 60 x [Total LA flow rate (g/min) / catalyst weight (g)].

As used herein, the term "Liquid Hourly Space Velocity" or "LHSV" in h$^{-1}$ is defined as 60 x [Total liquid flow rate (mL/min) / catalyst bed volume (mL)].

As used herein, the term "bio-based" material refers to a renewable material.

As used herein, the term "renewable material" refers to a material that is produced from a renewable resource.

As used herein, the term "renewable resource" refers to a resource that is produced via a natural process at a rate comparable to its rate of consumption (e.g., within a 100 year time frame). The resource can be replenished naturally, or via agricultural techniques. Non-limiting examples of renewable resources include plants (e.g., sugar cane, beets, corn, potatoes, citrus fruit, woody plants, lignocellulose, hemicellulose, and cellulosic waste), animals, fish, bacteria, fungi, and forestry products. These resources can be naturally occurring, hybrids, or genetically engineered organisms. Natural resources, such as crude oil, coal, natural gas, and peat, which take longer than 100 years to form, are not considered renewable resources. Because at least part of the material of the invention is derived from a renewable resource, which can sequester carbon dioxide, use of the material can reduce global warming potential and fossil fuel consumption.

As used herein, the term "petroleum-based" material refers to a material that is produced from fossil material, such as petroleum, natural gas, coal, etc.

II. Catalysts for the Conversion of Hydroxypropionic Acid or its Derivatives to Acrylic Acid or its Derivatives

Unexpectedly, it has been found that catalysts comprising a mixture of: 1) partially dehydrated dihydrogen monophosphates of monovalent cations in the amorphous state, 2) crystalline polyphosphates of polyvalent cations, and 3) non-phosphate salts of polyvalent cations (e.g. sulfates, tantalates) can dehydrate hydroxypropionic acid, hydroxypropionic acid derivatives, or mixtures thereof to acrylic acid, acrylic acid derivatives, or mixtures thereof with high: 1) yield and selectivity for acrylic acid, acrylic acid derivatives, or mixtures thereof, i.e., low amount and few side products; 2) efficiency, i.e., performance in short residence time; and 3) longevity. As a non limiting example, the amorphous state of said mixture of partially dehydrated dihydrogen monophosphates can be formed reversibly when crystalline phosphate salts (e.g. monophosphates, polyphosphates, or cyclophosphates) of monovalent cations with molar ratio of phosphorus to cations of about 1 are contacted with water at elevated water partial pressure and temperature. The applicants also found unexpectedly, that in order to dehydrate hydroxypropionic acid, hydroxypropionic acid derivatives, or mixtures thereof to acrylic acid, acrylic acid derivatives, or mixtures thereof, the dehydration catalyst of the present invention needs to be in the presence of sufficient water vapor, contrary to common belief in the art of performing dehydration reactions under dry conditions. Although not wishing to be bound by any theory, applicants hypothesize that the water vapor is required to avoid full dehydration of the dihydrogen monophosphate salts to condensed phosphates under operation conditions, maintaining the Brønsted acid sites that are required for the selective acid-catalyzed dehydration of hydroxypropionic acid and its derivatives to acrylic acid and its derivatives.

The dehydration catalysist consists essentially of one or more amorphous phosphate salts, one or more crystalline phosphate salts, and one or more non-phosphate salts and amorphous silica, wherein the amorphous phosphate salt is $KH_{2(1-x)}PO_{(4-x)}$, wherein the non-phosphate salt is $BaSO_4$, and the crystalline phosphate salt is $Ba_2P_2O_7$.

In the context of the present invention, a phosphate salt or a mixture of phosphate salts with more than 50 wt% of amorphous content (or less than 50 wt% of crystalline content) are considered amorphous phosphate salts. The amorphous content can be determined by any method known to those skilled in the art, such as, by way of example and not limitation, x-ray diffraction (XRD), infrared spectroscopy (IR), Raman spectroscopy, differential scanning calorimetry (DSC), or solid-state nuclear magnetic resonance (NMR) spectroscopy. As an illustration, in a method based on an XRD technique (see Figure 1), the separate crystalline ($I_C$) and amorphous ($I_A$) contributions on the X-ray scattering pattern are determined using a profile-fitting technique. This deconvolution of the scattering pattern into the separate contributions can be performed using Gaussian, Lorentzian, Voigt, or related functions known to those skilled in the art. Then, the amorphous content, $X_A$, is determined by calculating the ratio between the area of scattered intensity for the amorphous contribution ($I_A$) and the area of the total scattered intensity (crystalline plus amorphous contributions, $I_T = I_C + I_A$) for a defined Bragg angle range (e.g. $2\theta = 5°$ to $50°$, Cu-radiation $\lambda = 1.54059$ Å, in the context of the current invention), i.e.

$$X_A = \frac{I_A}{I_C + I_A} \times 100 \ wt\%$$

In another embodiment of the present invention, at least one of said one or more amorphous phosphate salts of said dehydration catalyst is a hydrated salt. In another embodiment of the present invention, at least one of said one or more crystalline phosphate salts is a hydrated salt. In another embodiment of the present invention, at least one of said one or more non-phosphate salts is a hydrated salt. A hydrated salt contains a specific number of water molecules per formula unit of the salt. Non limiting examples of hydrated salts are hemihydrated, monohydrated, sesquihydrated, dehydrated, trihydrated, tetrahydrated, pentahydrated, hexahydrated, heptahydrated, octahydrated, nonahydrated, nonahydrated, and decahydrated salts.

In the present invention, said one or more amor-

phous phosphate salts is $KH_{2(1-x)}PO_{(4-x)}$, wherein x is any real number equal to or greater than 0 and equal to or less than 1; said one or more crystalline phosphate salts is $Ba_2P_2O_7$; and said one or more non-phosphate compounds is $BaSO_4$.

In the present invention, said dehydration catalyst consists essentially of $KH_{2(1-x)}PO_{(4-x)}$, $BaSO_4$, $Ba_2P_2O_7$, and amorphous silica; wherein x is any real number equal to or greater than 0 and equal to or less than 1.

III. Catalyst Preparation Methods

A method of preparing the dehydration catalyst comprises contacting:

(a) a dehydration catalyst precursor mixture with
(b) a gas mixture comprising water vapor;

wherein the water partial pressure in said gas mixture is equal to or greater than the water partial pressure at the triple point of at least one of said one or more amorphous phosphate salt precursors; wherein said contacting step between said dehydration catalyst precursor mixture and said gas mixture is performed at a temperature equal to or greater than the temperature at the triple point of at least one of said one or more amorphous phosphate salt precursors; and wherein one or more amorphous phosphate salts are produced as a result of said one or more amorphous phosphate salt precursors being contacted with said water vapor.

In the context of the present invention, the triple point is the temperature and water partial pressure at which three phases: crystalline dihydrogen monophosphate or dihydrogen diphosphate salt, crystalline polyphosphate salt, and amorphous phosphate salt coexist in thermodynamic equilibrium. By way of example, and not limitation, the triple point can be located by determining the interception of two (out of three) phase boundary curves in the water partial pressure versus temperature phase equilibrium diagram (see Figure 2):

Curve A: phase boundary between i) crystalline dihydrogen monophosphate or crystalline dihydrogen diphosphate salt and ii) crystalline polyphosphate salt, at low temperatures and water partial pressures (e.g. below about 248 °C and 0.85 bar for potassium salts, below about 267 °C and 0.35 bar for cesium salts);
Curve B: phase boundary between i) crystalline polyphosphate salt and ii) amorphous phosphate salt at high temperatures and medium water partial pressures (e.g. above about 248 °C and 0.85 bar for potassium salts, above about 267 °C and 0.35 bar for cesium salts); and
Curve C: phase boundary between i) crystalline dihydrogen monophosphate or crystalline dihydrogen diphosphate salt and ii) amorphous phosphate salt at high temperatures and high water partial pressures.

The phase boundary curves can be determined by any method known to those skilled in the art, such as, by way of example and not limitation, in-situ x-ray diffraction (XRD), thermal analysis (e.g. thermogravimetric analysis, differential thermal analysis, and differential scanning calorimetry), Raman spectroscopy, infrared spectroscopy (IR), nuclear magnetic resonance (NMR) spectroscopy, or the methods described in Taninouchi, Y.-k., et al., J. Electrochem. Soc. 156:B572-B579 (2009); or Ikeda, A. and Haile, S. M., Solid State Ionics 2012, 213:63-71 (2012). As an illustration, in a method based on the in-situ XRD technique, a precursor phosphate salt is contacted at high temperature (e.g. 450 °C) with a gas stream consisting of an inert gas (e.g. nitrogen, helium, or air) and water vapor at a specific water partial pressure until equilibrium is achieved. Then, the temperature is gradually decreased while monitoring changes on x-ray diffraction patterns, until a phase transition is observed. The same procedure is repeated at different water partial pressures and the transition temperatures are recorded. The water partial pressures (in logarithmic scale) are plotted against the transition temperatures (in linear scale) and fitted to the Arrhenius equation ($log_{10}(P_{H_2O}) = A + B/T$). Finally, the triple point is calculated by determining the interception point between the two phase boundary curves (i.e. curve A and curve B in Figure 2).

IV. Methods of Producing Acrylic Acid, Acrylic Acid Derivatives, or Mixtures Thereof

The inventors have unexpectedly found that the method of dehydrating hydroxypropionic acid, hydroxypropionic acid derivatives, or mixtures thereof can produce high yield to and selectivity of acrylic acid, acrylic acid derivatives, or mixtures thereof when the dehydration catalyst is prepared according to the present invention and the dehydration reaction is operated under a water partial pressure of more than about 0.4 bar. Not wishing to be bound by theory, the inventors believe that the elevated water partial pressure enhances the catalyst activity due to the formation (or preservation) of Brønsted acid sites from less protonated catalyst precursors. Thus, the inventors have also unexpectedly found that the process of dehydrating hydroxypropionic acid can be more efficient in the presence of elevated water partial pressure than under low water partial pressure or atmospheric conditions usually preferred in the art.

A method for dehydrating hydroxypropionic acid, hydroxypropionic acid derivatives, or mixtures thereof to acrylic acid, acrylic acid derivatives, or mixtures thereof is provided. In one embodiment of the present invention, said hydroxypropionic acid is selected from the group consisting of lactic acid (2-hydroxypropionic), 3-hydroxypropionic acid, and mixtures thereof; and said hydroxypropionic acid derivatives are selected from the group consisting of lactic acid derivatives, 3-hydroxypropionic acid derivatives, and mixtures thereof. In another embodiment of the present invention, said hydroxypropionic acid is lactic acid and said hydroxypropionic acid derivatives are lactic acid derivatives.

Lactic acid can be D-lactic acid, L-lactic acid, or mixture thereof. Lactic acid derivatives can be metal or ammonium salts of lactic acid, alkyl esters of lactic acid, lactic acid oligomers, cyclic di-esters of lactic acid, lactic acid anhydride, 2-alkoxypropionic acids or their alkyl esters, 2-aryloxypropionic acids or their alkyl esters, 2-acyloxypropionic acids or their alkyl esters, or a mixture thereof. Non limiting examples of metal salts of lactic acid are sodium lactate, potassium lactate, and calcium lactate. Non limiting examples of alkyl esters of lactic acid are methyl lactate, ethyl lactate, butyl lactate, 2-ethylhexyl lactate, and mixtures thereof. A non limiting example of cyclic di-esters of lactic acid is dilactide. Non limiting examples of 2-alkoxypropionic acids are 2-methoxypropionic acid and 2-ethoxypropionic acid. A non limiting example of 2-aryloxypropionic acid is 2-phenoxypropionic acid. A non limiting example of 2-acyloxypropionic acid is 2-acetoxypropionic acid. In one embodiment of the present invention, the lactic acid derivative is methyl lactate. Methyl lactate can be neat or in a solution with water, methanol, or mixtures thereof.

3-hydroxypropionic acid derivatives can be metal or ammonium salts of 3-hydroxypropionic acid, alkyl esters of 3-hydroxypropionic acid, 3-hydroxypropionic acid oligomers, 3-alkoxypropionic acids or their alkyl esters, 3-aryloxypropionic acids or their alkyl esters, 3-acyloxypropionic acids or their alkyl esters, or a mixture thereof. Non limiting examples of metal salts of 3-hydroxypropionic acid are sodium 3-hydroxypropionate, potassium 3-hydroxypropionate, and calcium 3-hydroxypropionate. Non limiting examples of alkyl esters of hydroxypropionic acid are methyl 3-hydroxypropionate, ethyl 3-hydroxypropionate, butyl 3-hydroxypropionate, 2-ethylhexyl 3-hydroxypropionate, and mixtures thereof. Non limiting examples of 3-alkoxypropionic acids are 3-methoxypropionic acid and 3-ethoxypropionic acid. A non limiting example of 3-aryloxypropionic acid is 3-phenoxypropionic acid. A non limiting example of 3-acyloxypropionic acid is 3-acetoxypropionic acid.

Acrylic acid derivatives can be metal or ammonium salts of acrylic acid, alkyl esters of acrylic acid, acrylic acid oligomers, or mixtures thereof. Non limiting examples of metal salts of acrylic acid are sodium acrylate, potassium acrylate, and calcium acrylate. Non limiting examples of alkyl esters of acrylic acid are methyl acrylate, ethyl acrylate, butyl acrylate, 2-ethylhexyl acrylate, or mixtures thereof.

In the present invention, a method of making acrylic acid, metal or ammonium salts of acrylic acid, alkyl esters of acrylic acid, acrylic acid oligomers, or mixtures thereof comprises contacting the following compositions under conditions including a water partial pressure and a temperature:

a gas mixture comprising:

a) hydroxypropionic acid, metal or ammonium salts of lactic acid, alkyl esters of lactic acid, lactic acid oligomers, cyclic di-esters of lactic acid, lactic acid anhydride, 2-alkoxypropionic acids or their alkyl esters, 2-aryloxypropionic acids or their alkyl esters, 2-acyloxypropionic acids or their alkyl esters, metal or ammonium salts of 3-hydroxypropionic acid, alkyl esters of 3-hydroxypropionic acid, 3-hydroxypropionic acid oligomers, 3-alkoxypropionic acids or their alkyl esters, 3-aryloxypropionic acids or their alkyl esters, 3-acyloxypropionic acids or their alkyl esters, or mixtures thereof; and

b) water vapor; with

a dehydration catalyst consisting essentially of one or more amorphous phosphate salts, one or more crystalline phosphate salts, and one or more non-phosphate salts and amorphous silica, wherein the amorphous phosphate salt is $KH_{2(1-x)}PO_{(4-x)}$, wherein the non-phosphate salt is $BaSO_4$, and the crystalline phosphate salt is $Ba_2P_2O_7$, wherein x is any real number equal to or greater than 0 and equal to or less than 1;

wherein the water partial pressure during said contacting step in said gas mixture is equal to or greater than the water partial pressure at the triple point of said one or more amorphous phosphate salts; wherein said contacting step is performed at a temperature equal to or greater than the temperature at the triple point of said one or more amorphous phosphate salts; and whereby said acrylic acid, metal or ammonium salt of acrylic acids, alkyl esters of acrylic acid, acrylic acid oligomers, or mixtures thereof is produced as a result of said water vapor and said hydroxypropionic acid, metal or ammonium salts of lactic acid, alkyl esters of lactic acid, lactic acid oligomers, cyclic di-esters of lactic acid, lactic acid anhydride, 2-alkoxypropionic acids or their alkyl esters, 2-aryloxypropionic acids or their alkyl esters, 2-acyloxypropionic acids or their alkyl esters, metal or ammonium salts of 3-hydroxypropionic acid, alkyl esters of 3-hydroxypropionic acid, 3-hydroxypropionic acid oligomers, 3-alkoxypropionic acids or their alkyl esters, 3-aryloxypropionic acids or their alkyl esters, 3-acyloxypropionic acids or their alkyl esters, or mixtures thereof being contacted with said dehydration catalyst.

In another embodiment of the present invention, said gas mixture further comprises an essentially chemically inert gas. In the context of the present invention, an essentially chemically inert gas is any gas that is essentially chemically inert to said hydroxypropionic acid, hydroxypropionic acid derivatives, or mixtures thereof, but not necessarily to said dehydration catalyst or said dehydration catalyst precursor mixture. Non limiting examples of essentially chemically inert gases are nitrogen, helium, argon, carbon dioxide, carbon monoxide, air, water vapor, and mixtures thereof. In another embodiment of the present invention, said essentially chemically inert gas comprises nitrogen. In yet another embodiment of the present invention, said essentially chemically inert gas consists essentially of nitrogen.

In the context of the present invention, "contacting" refers to the action of bringing said hydroxypropionic acid, hydroxypropionic acid derivatives, or mixtures thereof in close proximity to the surface of said dehydration catalyst. The hydroxypropionic acid, hydroxypropionic acid derivatives, or mixtures thereof must contact the surface of the dehydration catalyst at a rate that is slow enough for the dehydration reaction to occur, yet fast enough to avoid the degradation of hydroxypropionic acid, acrylic acid, or their derivatives to undesirable products at the temperature of said contacting step. Several parameters can be used to describe the rate of said contacting step, such as, by way of example and not limitation, WHSV, GHSV, LHSV, and weight velocity per unit of accessible catalyst surface area (WVSA) that can be calculated as the ratio of WHSV and the dehydration catalyst specific surface area (SA), (WVSA = WHSV/SA); with units: $g/m^2 \cdot h$; where g refer to g of hydroxypropionic acid, hydroxypropionic acid derivatives, or mixtures thereof. A number of methods, based on the adsorption of an inert gas, can be used to determine the accessible surface area, including, but not limited to, the static volumentric and gravimetric methods and the dynamic method that are well-known by those skilled in the art.

In one embodiment, the hydroxypropionic acid, hydroxypropionic acid derivatives, or mixtures thereof contact the dehydration catalyst at a WVSA between about $10^4$ g·m$^{-2}$·h$^{-1}$ and about $10^{-4}$ g·m$^{-2}$·h$^{-1}$.

In one embodiment, the hydroxypropionic acid, hydroxypropionic acid derivatives, or mixtures thereof contact the dehydration catalyst at a WHSV between about 0.02 h$^{-1}$ and about 10 h$^{-1}$.

When hydroxypropionic acid, hydroxypropionic acid derivatives, or mixtures thereof are in the gas phase during said contacting step with said dehydration catalyst, and in another embodiment, the gas mixture comprising hydroxypropionic acid, hydroxypropionic acid derivatives, or mixtures thereof contacts the dehydration catalyst or the dehydration catalyst precursor mixture at a GHSV between about 720 h$^{-1}$ and about 36,000 h$^{-1}$.

When hydroxypropionic acid, hydroxypropionic acid derivatives, or mixtures thereof are in the gas phase during said contacting step with said dehydration catalyst, and in one embodiment, the concentration of hydroxypropionic acid, hydroxypropionic acid derivatives, or mixtures thereof before the dehydration reaction and based on the total moles in the gas mixture (calculated under STP conditions) is between about 0.5 mol% and about 50 mol%.

In one embodiment, the temperature during said contacting step with the dehydration catalyst is greater than about 150 °C. In another embodiment of the present invention, the temperature during said contacting step with the dehydration catalyst is greater than about 250 °C.

In the present invention, the temperature during said contacting step with the dehydration catalyst is equal to or greater than the temperature at the triple point of at least one of said one or more amorphous phosphate salts. In another embodiment, the temperature during said contacting step with the dehydration catalyst is equal to or greater than the lowest triple point temperature of said one or more amorphous phosphate salts. In another embodiment, the temperature during said contacting step with the dehydration catalyst is equal to or greater than the highest triple point temperature of said one or more amorphous phosphate salts. In another embodiment, the temperature during said contacting step with the dehydration catalyst is equal to or greater than the average temperature between the lowest triple point temperature and the highest triple point temperature of said one or more amorphous phosphate salts. In another embodiment, the temperature during said contacting step with the dehydration catalyst is at least 10 °C greater than the temperature at the triple point of at least one of said one or more amorphous phosphate salts. In another embodiment, the temperature during said contacting step with the dehydration catalyst is at least 50 °C greater than the temperature at the triple point of at least one of said one or more amorphous phosphate salts. In another embodiment, the temperature during said contacting step with the dehydration catalyst is at least 100 °C greater than the temperature at the triple point of at least one of said one or more amorphous phosphate salts.

In another embodiment, said water partial pressure during said contacting step with the dehydration catalyst is equal to or greater than about 0.4 bar. In another embodiment of the present invention, said water partial pressure during said contacting step with the dehydration catalyst is equal to or greater than about 0.8 bar. In another embodiment, said water partial pressure during said contacting step with the dehydration catalyst is equal to or greater than about 4 bar. In another embodiment, said water partial pressure during said contacting step with the dehydration catalyst is between about 5 bar and about 35 bar.

In the present invention, the water partial pressure during said contacting step with the dehydration catalyst is equal to or greater than the water partial pressure at the triple point of at least one of said one or more amorphous phosphate salts. In another embodiment, the water partial pressure during said contacting step with the dehydration catalyst is equal to or greater than the lowest triple point water partial pressure of said one or more amorphous phosphate salts. In another embodiment, the water partial pressure during said contacting step with the dehydration catalyst is equal to or greater than the highest triple point water partial pressure of said one or more amorphous phosphate salts. In another embodiment, the water partial pressure during said contacting step with the dehydration catalyst is equal to or greater than the average water partial pressure between the lowest triple point water partial pressure and the highest triple point water partial pressure of said one or more amorphous phosphate salts. In one embodiment, the water partial pressure during said contacting step with the dehydration catalyst is at least 1 bar greater than the water partial pressure at the triple point of at least one of said one or more amorphous phosphate salts . In one embodiment, the water partial pressure during said contacting step with the dehydration catalyst is at least 2 bar greater than the water partial pressure at the triple point of at least one of said one or more amorphous phosphate salts . In one embodiment, the water partial pressure during said contacting step with the dehydration catalyst is at least 5 bar greater than the water partial pressure at the triple point of at least one of said one or more amorphous phosphate salts.

The contacting step with the dehydration catalyst mixture can be performed under vacuum, at atmospheric pressure, or at higher than atmospheric pressure. In one embodiment, the contacting step with the dehydration catalyst is performed under a total pressure of at least about 1 bar. In another, the contacting step with the dehydration catalyst is performed under a total pressure between about 5 bar and about 40 bar. In another embodiment, the contacting step with the dehydration catalyst is performed under a total pressure between about 10 bar and about 35 bar. In yet another embodiment, the contacting step with the dehydration catalyst is performed under a total pressure of about 25 bar.

When hydroxypropionic acid, hydroxypropionic acid derivatives, or mixtures thereof are in the gas phase, and in another embodiment, the contacting step with the dehydration catalyst is performed in a catalytic reactor with the gas mixture flowing down, flowing up, or flowing horizontally. In another embodiment , the contacting step with the dehydration catalyst is performed in a catalytic reactor with the gas mixture flowing down. Also, the contacting step with the dehydration catalyst can be done in a batch form. In another embodiment, the dehydration catalyst is suspended in an essentially chemically inert liquid. The contacting step with the dehydration catalyst can be performed by using different catalytic reactors known to those skilled in the art, such as, by way of example and not limitation, static reactor, stirred reactor, recirculation reactor, packed-bed flow reactor, and combinations thereof.

In one embodiment, hydroxypropionic acid, hydroxypropionic acid derivatives, or mixtures thereof and water vapor contact the dehydration catalyst in a catalytic reactor with an interior surface material selected from the group consisting of amorphous silica, quartz, other silicon oxides, borosilicate glass, silicon, and mixtures thereof. In another embodiment, hydroxypropionic acid, hydroxypropionic acid derivatives, or mixtures thereof and water vapor contact the dehydration catalyst in a catalytic reactor with an interior surface material selected from the group consisting of amorphous silica, quartz, borosilicate glass, and mixtures thereof. In another embodiment, hydroxypropionic acid, hydroxypropionic acid derivatives, or mixtures thereof and water vapor contact the dehydration catalyst in a catalytic reactor with an interior surface material consisting essentially of borosilicate glass.

The acrylic acid mixture comprising acrylic acid, acrylic acid derivatives, or mixtures thereof produced in said contacting step with the dehydration catalyst is cooled to give a liquid acrylic acid composition as the product stream. The time required to cool the acrylic acid mixture must be controlled to reduce acrylic acid polymerization or decomposition to ethylene. In one embodiment, the residence time of the acrylic acid mixture in the cooling step is less than about 30 s. In one embodiment, the residence time of the acrylic acid mixture in the cooling step is between about 0.1 s and about 10 s.

The liquid acrylic acid composition comprising acrylic acid, acrylic acid derivatives, or mixtures thereof produced according with the present invention can be purified using some or all of the processes of extraction, drying, distilling, cooling, partial melting, and decanting described in US20130274518A1 to produce crude and glacial acrylic acid. After purification, the crude and glacial acrylic acid can be polymerized to produce a superabsorbent polymer using processes that are similar to those described in US20130274697A1.

In one embodiment, said crude acrylic acid is esterified with an alcohol to produce an acrylate monomer. Non-limiting examples of alcohols are methanol, ethanol, butanol (n-butyl alcohol), 2-ethyl hexanol, isobutanol, tert-butyl alcohol, hexyl alcohol, octyl alcohol, isooctyl alcohol, lauryl alcohol, propyl alcohol, isopropyl alcohol, hydroxyethyl alcohol, hydroxypropyl alcohol, and polyols, such as hydroxyalkyl and alkylalkanolamine. In another embodiment, said crude acrylic acid is esterified with methanol, ethanol, n-butyl alcohol, or 2-ethyl hexanol to produce methyl acrylate monomer, ethyl acrylate monomer, n-butyl acrylate monomer, or 2-ethylhexyl acrylate monomer, respectively. In yet another embodiment, said methyl acrylate monomer, ethyl acrylate monomer, n-butyl acrylate monomer, or 2-ethylhexyl acrylate monomer is polymerized to produce methyl acrylate polymer, ethyl acrylate polymer, n-butyl acrylate polymer, or 2-ethylhexyl acrylate polymer, respectively. In even yet another embodiment, said methyl acrylate monomer, ethyl acrylate monomer, n-butyl acrylate monomer, or 2-ethylhexyl acrylate monomer is co-polymerized with other monomer to produce methyl acrylate co-polymer, ethyl acrylate co-polymer, n-butyl acrylate co-polymer, or 2-ethylhexyl acrylate co-polymer, respectively. Non-limiting examples of other monomers are vinyl acetate and ethylene. In one embodiment, said methyl acrylate polymer, ethyl acrylate polymer, n-butyl acrylate polymer, or 2-ethylhexyl acrylate polymer is blended with methyl methacrylate (MMA) to produce blends of MMA and methyl acrylate polymer, blends of MMA and ethyl acrylate polymer, blends of MMA and n-butyl acrylate polymer, or blends of MMA and 2-ethylhexyl acrylate polymer, respectively. Non-limiting applications of polymers, co-polymers, or blends are in surface coatings, paints, resins, adhesives, plastics, and dispersions. In another embodiment, said alcohol is bio-based alcohol. In yet another embodiment, said other monomer is bio-based monomer. In even yet another embodiment, said MMA is bio-based MMA.

In one embodiment, the method of making acrylic acid, acrylic acid derivatives, or mixtures thereof comprises contacting said hydroxypropionic acid, hydroxypropionic acid derivatives, and mixture thereof and said water vapor with said dehydration catalyst under conditions sufficient to produce acrylic acid, acrylic acid derivatives, or mixtures thereof in a yield of at least 50%. In another embodiment, the method comprises contacting said hydroxypropionic acid, hydroxypropionic acid derivatives, and mixture thereof and said water vapor with said dehydration catalyst under conditions are sufficient to produce acrylic acid, acrylic acid derivatives, or mixtures thereof in a yield of at least about 70%. In another embodiment, the method comprises contacting said hydroxypropionic acid, hydroxypropionic acid derivatives, and mixture thereof and said water vapor with said dehydration catalyst under conditions are sufficient to produce acrylic acid, acrylic acid derivatives, or mixtures thereof in a yield of at least about 80%. In another embodiment, the method conditions are sufficient to produce acrylic acid, acrylic acid derivatives, or mixtures thereof with a selectivity of at least about 50%. In another embodiment, the method conditions are sufficient to produce acrylic acid, acrylic acid derivatives, or mixtures thereof with a selectivity of at least about 70%. In another embodiment, the method conditions are sufficient to produce acrylic acid, acrylic acid derivatives, or mixtures thereof with a selectivity of at least about 80%. In another embodiment, the method conditions are sufficient to produce acrylic acid, acrylic acid derivatives, or mixtures thereof with propionic acid as an impurity, wherein the propionic acid selectivity is less than about 5%. In another embodiment, the method conditions are sufficient to produce acrylic acid, acrylic acid derivatives, or mixtures thereof with propionic acid as an impurity, wherein the propionic acid selectivity is less than about 1%. In another embodiment, the method conditions are sufficient to produce acrylic acid, acrylic acid derivatives, or mixtures thereof with a conversion of said hydroxypropionic acid, hydroxypropionic acid derivatives, or mixtures thereof of more than about 50%. In another embodiment, the method conditions are sufficient to produce acrylic acid, acrylic acid derivatives, or mixtures thereof with a conversion of said hydroxypropionic acid, hydroxypropionic acid derivatives, or mixtures thereof of more than about 80%.

Among the benefits attainable by the foregoing embodiments is the low yield of side products. In one embodiment, the conditions are sufficient to produce propionic acid in a yield of less than about 5% from hydroxypropionic acid. In another embodiment, the conditions are sufficient to produce propionic acid in a yield of less than about 1%, from hydroxypropionic acid. In one embodiment, the conditions are sufficient to produce each of acetic acid, pyruvic acid, 1,2-propanediol, hydroxyacetone, acrylic acid dimer, and 2,3-pentanedione in a yield of less than about 2% from hydroxypropionic acid present in the gaseous mixture. In another embodiment, the conditions are sufficient to produce each of acetic acid, pyruvic acid, 1,2-propanediol, hydroxyacetone, acrylic acid dimer, and 2,3-pentanedione in a yield of less than about 0.5%, from hydroxypropionic acid present in the gaseous mixture. In one embodiment, the conditions are sufficient to produce acetaldehyde in a yield of less than about 8% from hydroxypropionic acid present in the gaseous mixture. In another embodiment, the conditions are sufficient to produce acetaldehyde in a yield of less than about 4% from hydroxypropionic acid present in the gaseous mixture. In another embodiment, the conditions are sufficient to produce acetaldehyde in a yield of less than about 3%, from hydroxypropionic acid present in the gaseous mixture. These yields are believed to be, heretofore, unattainably low. Yet, these benefits are indeed achievable as further evidenced in the Examples set out below.

V. Examples

EXAMPLE 1

Potassium phosphate monobasic ($KH_2PO_4$), barium phosphate dibasic ($BaHPO_4$), barium sulfate ($BaSO_4$), and amorphous silicon oxide ($SiO_2$) are combined and ground together using a planetary ball mill to obtain a fine solid. The solid is transferred to a glass beaker and calcined at a temperature between about 450°C and about 450°C for 4 h to 12 h. The calcined solid is ground and sieved to obtain a dehydration catalyst precursor mixture with particle size between 106 $\mu$m and 212 $\mu$m. The dehydration catalyst precursor mixture is mainly composed of T-$(KPO_3)_n$, $Ba_2P_2O_7$, $BaSO_4$, and amorphous SiO2.

The dehydration catalyst precursor mixture prepared as described above is tested for the conversion of lactic acid to acrylic acid. A glass-lined stainless steel tube is packed with glass wool at the bottom, followed by dehydration catalyst precursor mixture in the middle and free space at the top. The tube is placed inside an aluminum block and a clam shell furnace in a down-flow arrangement and the bottom of the reactor is connected to a catch tank using fused silica lined stainless steel tubing. The reactor is purged by flowing $N_2$ gas (45 mL/min) at 360 psig (25 barg) (36000 KPa gauge). Then, the reactor is heated until a final temperature of about 375 °C (tube wall temperature) is reached. A liquid solution of lactic acid in water (20.0 wt%) is fed at the top of the reactor at 0.045 mL/min though polyetheretherketone (PEEK™) tubing using a pump. Before contacting the dehydration catalyst, the gas phase concentrations are: nitrogen: 47.9 mol%; lactic acid: 2.5 mol%; and water: 49.6 mol% and the water partial pressure is 186 psi (12.8 bar) (18600 KPa). After contacting the dehydration catalyst, the reactor effluent is cooled and the liquid containing acrylic acid is collected in the catch tank. The uncondensed gas effluents are discharged.

The foregoing description is given for clearness of understanding only, and no unnecessary limitations should be understood therefrom, as modifications within the scope of the invention may be apparent to those having ordinary skill in the art.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

While particular embodiments of the present invention have been illustrated and described, it would be obvious to those skilled in the art that various other changes and modifications can be made without departing from the scope of the invention, which is defined by the appended claims.

## Claims

1. A method of making acrylic acid, metal or ammonium salts of acrylic acid, alkyl esters of acrylic acid, acrylic acid oligomers, or mixtures thereof comprising contacting the following compositions under conditions including a water partial pressure and a temperature:

   a gas mixture comprising:

   a) hydroxypropionic acid, metal or ammonium salts of lactic acid, alkyl esters of lactic acid, lactic acid oligomers, cyclic di-esters of lactic acid, lactic acid anhydride, 2-alkoxypropionic acids or their alkyl esters, 2-aryloxypropionic acids or their alkyl esters, 2-acyloxypropionic acids or their alkyl esters, metal or ammonium salts of 3-hydroxypropionic acid, alkyl esters of 3-hydroxypropionic acid, 3-hydroxypropionic acid oligomers, 3-alkoxypropionic acids or their alkyl esters, 3-aryloxypropionic acids or their alkyl esters, 3-acyloxypropionic acids or their alkyl esters, or mixtures thereof; and
   b) water vapor; with

   a dehydration catalyst consisting essentially of one or more amorphous phosphate salts, one or more crystalline phosphate salts, and one or more non-phosphate salts and amorphous silica, wherein the amorphous phosphate salt is $KH_{2(1-x)}PO_{(4-x)}$, wherein the non-phosphate salt is $BaSO_4$, and the crystalline phosphate salt is $Ba_2P_2O_7$, wherein x is any real number equal to or greater than 0 and equal to or less than 1; wherein the water partial pressure during said contacting step in said gas mixture is equal to or greater than the water partial pressure at the triple point of said one or more amorphous phosphate salts; wherein said contacting step is performed at a temperature equal to or greater than the temperature at the triple point of said one or more amorphous phosphate salts; and whereby said acrylic acid, metal or ammonium salt of acrylic acids, alkyl esters of acrylic acid, acrylic acid oligomers, or mixtures thereof is produced as a result of said water vapor and said hydroxypropionic acid, metal or ammonium salts of lactic acid, alkyl esters of lactic acid, lactic acid oligomers, cyclic di-esters of lactic acid, lactic acid anhydride, 2-alkoxypropionic acids or their alkyl esters, 2-aryloxypropionic acids or their alkyl esters, 2-acyloxypropionic acids or their alkyl esters, metal or ammonium salts of 3-hydroxypropionic acid, alkyl esters of 3-hydroxypropionic acid, 3-hydroxypropionic acid oligomers, 3-alkoxypropionic acids or their alkyl esters, 3-aryloxypropionic acids or their alkyl esters, 3-acyloxypropionic acids or their alkyl esters, or mixtures thereof being contacted with said dehydration catalyst.

2. The method of claim 1, wherein the dehydration catalyst consists essentially of $KH_2PO_4$, $BaSO_4$, $Ba_2P_2O_7$, and amorphous silica

3. The method of claim 1, wherein the water partial pressure during said contacting step in said gas mixture is equal to or greater than about 0.8 bar; and wherein said contacting step is performed at a temperature equal to or greater than about 250 °C.

## Patentansprüche

1. Verfahren zum Herstellen von Acrylsäure, Metalloder Ammoniumsalzen von Acrylsäure, Alkylestern von Acrylsäure, Acrylsäure-Oligomeren oder Mischungen davon, umfassend das Inkontaktbringen der folgenden Zusammensetzungen unter Bedingungen, einschließlich eines Wasserpartialdrucks und einer Temperatur:

   einer Gasmischung, umfassend:

   a) Hydroxypropionsäure, Metall- oder Ammoniumsalze von Milchsäure, Alkylester von Milchsäure, Milchsäure-Oligomere, cyclische Diester von Milchsäure, Milchsäureanhydrid, 2-Alkoxypropionsäuren oder deren Alkylester, 2-Aryloxypropionsäuren oder deren Alkylester, 2-Acyloxypropionsäuren oder deren Alkylester, Metall- oder Ammoniumsalze von 3-Hydroxypropionsäure, Alkylester von 3-Hydroxypropionsäure, 3-Hydroxypropionsäure-Oligomere, 3-Alkoxypropionsäuren oder deren Alkylester, 3-Aryloxypropionsäuren oder deren Alkylester, 3-Acyloxypropionsäuren oder deren Alkylester oder Mischungen davon; und

b) Wasserdampf; wobei

ein Dehydratationskatalysator, der im Wesentlichen aus einem oder mehreren amorphen Phosphatsalzen, einem oder mehreren kristallinen Phosphatsalzen und einem oder mehreren Nichtphosphatsalzen und amorphem Silica besteht, wobei das amorphe Phosphatsalz $KH_{2(1-x)}PO_{(4-x)}$ ist, wobei das Nichtphosphatsalz $BaSO_4$ ist und das kristalline Phosphatsalz $Ba_2P_2O_7$ ist, wobei x eine beliebige reelle Zahl ist, die gleich oder größer als 0 und gleich oder kleiner als 1 ist; wobei der Wasserpartialdruck während des Schritt des Inkontaktbringens in der Gasmischung gleich oder größer als der Wasserpartialdruck an dem Tripelpunkt des einen oder der mehreren amorphen Phosphatsalze ist; wobei der Schritt des Inkontaktbringens bei einer Temperatur, die gleich oder höher als die Temperatur an dem Tripelpunkt des einen oder der mehreren amorphen Phosphatsalze ist, durchgeführt wird; und wobei die Acrylsäure, das Metall- oder Ammoniumsalz von Acrylsäuren, die Alkylester von Acrylsäure, die Acrylsäure-Oligomere oder Mischungen davon als ein Ergebnis von Wasserdampf und der Hydroxypropionsäure, Metall- oder Ammoniumsalzen von Milchsäure, Alkylestern von Milchsäure, Milchsäure-Oligomeren, zyklischen Diestern von Milchsäure, Milchsäureanhydrid, 2-Alkoxypropionsäuren oder deren Alkylester, 2-Aryloxypropionsäuren oder deren Alkylester, 2-Acyloxypropionsäuren oder deren Alkylester, Metall- oder Ammoniumsalzen von 3-Hydroxypropionsäure, Alkylestern von 3-Hydroxypropionsäure, 3-Hydroxypropionsäure-Oligomeren, 3-Alkoxypropionsäuren oder deren Alkylester, 3-Aryloxypropionsäuren oder deren Alkylester, 3-Acyloxypropionsäuren oder deren Alkylester oder Mischungen davon, die mit dem Dehydratationskatalysator in Kontakt gebracht werden.

2. Verfahren nach Anspruch 1, wobei der Dehydratationskatalysator im Wesentlichen aus $KH_2PO_4$, $BaSO_4$, $Ba_2P_2O_7$ und amorphem Silica besteht.

3. Verfahren nach Anspruch 1, wobei der Wasserpartialdruck während des Schritt des Inkontaktbringens in der Gasmischung gleich oder größer als etwa 0,8 bar ist; und wobei der Schritt des Inkontaktbringens bei einer Temperatur, die gleich oder größer als etwa 250 °C ist, durchgeführt wird.

**Revendications**

1. Procédé de fabrication d'acide acrylique, de sels métalliques ou d'ammonium d'acide acrylique, d'esters d'alkyle d'acide acrylique, d'oligomères d'acide acrylique, ou de mélanges de ceux-ci, comprenant la mise en contact des compositions suivantes dans des conditions comportant une pression partielle d'eau et une température :

un mélange gazeux comprenant :

a) acide hydroxypropionique, sels métalliques ou d'ammonium d'acide lactique, esters d'alkyle d'acide lactique, oligomères d'acide lactique, di-esters cycliques d'acide lactique, anhydride d'acide lactique, acides 2-alcoxypropioniques ou leurs esters d'alkyle, acides 2-aryloxypropioniques ou leurs esters d'alkyle, acides 2-acyloxypropioniques ou leurs esters d'alkyle, sels métalliques ou d'ammonium d'acide 3-hydroxypropionique, esters d'alkyle d'acide 3-hydroxypropionique, oligomères d'acide 3-hydroxypropionique, acides 3-alcoxypropioniques ou leurs esters d'alkyle, acides 3-aryloxypropioniques ou leurs esters d'alkyle, acides 3-acyloxypropioniques ou leurs esters d'alkyle, ou mélanges de ceux-ci ; et

b) vapeur d'eau ; avec

un catalyseur de déshydratation constitué sensiblement d'un ou plusieurs sels de phosphate amorphes, d'un ou plusieurs sels de phosphate cristallins, et d'un ou plusieurs sels non de phosphate et de silice amorphe, dans lequel le sel de phosphate amorphe est $KH_{2(1-x)}PO_{(4-x)}$, dans lequel le sel non de phosphate est $BaSO_4$, et le sel de phosphate cristallin est $Ba_2P_2O_7$, dans lequel x est un nombre réel quelconque supérieur ou égal à 0 et inférieur ou égal à 1 ; dans lequel la pression partielle de l'eau au cours de ladite étape de mise en contact dans ledit mélange gazeux est supérieure ou égale à la pression partielle de l'eau au point triple dudit ou desdits sels de phosphate amorphes ; dans lequel ladite étape de mise en contact est effectuée à une température supérieure ou égale à la température au point triple dudit ou desdits sels de phosphate amorphes ; et moyennant quoi ledit acide acrylique, ledit sel métallique ou d'ammonium d'acides acryliques, lesdits esters d'alkyle d'acide acrylique, lesdits oligomères d'acide acrylique, ou mélanges de ceux-ci sont produits à la suite de la mise en contact de ladite vapeur d'eau et dudit acide hydroxypropionique, desdits sels métalliques ou d'ammonium d'acide lactique, desdits esters d'alkyle d'acide lactique, desdits oligomères d'acide lactique, desdits di-esters cycliques d'acide lactique, dudit anhydride d'acide lactique, desdits acides 2-alcoxypropioniques ou leurs esters d'alkyle, desdits acides 2-aryloxypropioniques ou leurs esters d'alkyle, desdits acides 2-acyloxypropioniques ou leurs esters d'alkyle, desdits sels métalliques ou d'ammonium d'acide 3-hydroxypropionique, desdits esters d'alkyle d'acide 3-hydroxypropionique, desdits oligomères d'acide 3-hydroxypropionique, desdits acides 3-alcoxypropioniques ou leurs esters d'alkyle, desdits acides 3-aryloxypropioniques ou leurs esters d'alkyle, desdits acides 3-acyloxypropioniques ou leurs esters d'alkyle, ou mélanges de ceux-ci, avec ledit catalyseur de déshydratation.

2. Procédé selon la revendication 1, dans lequel le catalyseur de déshydratation est constitué sensiblement de $KH_2PO_4$, $BaSO_4$, $Ba_2P_2O_7$ et silice amorphe

3. Procédé selon la revendication 1, dans lequel la pression partielle de l'eau pendant ladite étape de mise en contact dans ledit mélange gazeux est supérieure ou égale à environ 0,8 bar ; et dans lequel ladite étape de mise en contact est effectuée à une température supérieure ou égale à environ 250 °C.

Fig. 1

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4786756 A
- CN 200910054519
- US 2013274515 A1
- US 2859240 A
- US 2015105584 A1
- US 20130274518 A1
- US 20130274697 A1

### Non-patent literature cited in the description

- **KIRK-OTHMER.** Encyclopedia of Chemical Technology. John Wiley & Sons, Inc, 2004, vol. 1, 342-369
- **HONG et al.** *Appl. Catal. A: General,* 2011, vol. 396, 194-200
- **GUNTER et al.** *J. Catalysis,* 1994, vol. 148, 252-260
- **TAM et al.** *Ind. Eng. Chem. Res.,* 1999, vol. 38, 3873-3877
- **JINFENG ZHANG et al.** Catalytic dehydration of lactic acid to acrylic acid over sulfate catalysts. *Canadian Journal of Chemical Engineering,* 01 December 2008, vol. 86, ISSN 0008-4034, 1047-1053
- Evaluation of Catalysts and Optimization of Reaction Conditions for the Dehydration of Methyl Lactate to Acrylates. **ZHANG J et al.** CHINESE JOURNAL OF CHEMICAL ENGINEERING. CHEMICAL INDUSTRY PRESS, 01 April 2008, vol. 16, 263-269
- **TANINOUCHI, Y.-K. et al.** *J. Electrochem. Soc.,* 2009, vol. 156, B572-B579
- **IKEDA, A ; HAILE, S. M.** *Solid State Ionics,* 2012, vol. 213, 63-71